(19) Europäisches Patentamt
European Patent Office
Office européen des brevets

(11) EP 1 122 238 A1

(12) **EUROPEAN PATENT APPLICATION**
published in accordance with Art. 158(3) EPC

(43) Date of publication:
**08.08.2001 Bulletin 2001/32**

(21) Application number: **99947876.1**

(22) Date of filing: **13.10.1999**

(51) Int Cl.[7]: **C07C 69/734**, C07C 255/13, C07F 7/08, A01N 37/38

(86) International application number:
**PCT/JP99/05647**

(87) International publication number:
**WO 00/21915 (20.04.2000 Gazette 2000/16)**

(84) Designated Contracting States:
**AT BE CH CY DE DK ES FI FR GB GR IE IT LI LU MC NL PT SE**

(30) Priority: **14.10.1998 JP 29165398**

(71) Applicant: **Meiji Seika Kaisha, Ltd.**
**Tokyo 104-8002 (JP)**

(72) Inventors:
- **KURIHARA, Hiroshi,**
  **Pharmaceutical Research Center**
  **Yokohama-shi, Kanagawa 222-8567 (JP)**
- **YAMAMOTO, Kazumi,**
  **Pharmaceutical Research Center**
  **Yokohama-shi, Kanagawa 222-8567 (JP)**
- **TERAOKA, Takeshi,**
  **Pharmaceutical Research Center**
  **Yokohama-shi, Kanagawa 222-8567 (JP)**
- **OYAMA, Kazuhiko,**
  **Pharmaceutical Research Center**
  **Yokohama-shi, Kanagawa 222-8567 (JP)**

(74) Representative: **Rickard, Timothy Mark Adrian**
**Elkington and Fife,**
**Prospect House,**
**8 Pembroke Road**
**Sevenoaks, Kent TN13 1XR (GB)**

# (54) METHYL ACETATE DERIVATIVES AND PESTICIDE COMPOSITIONS CONTAINING THE SAME AS THE ACTIVE INGREDIENT

(57) The present invention relates to a methyl acetate derivative shown in the following formula (1), and a pesticide composition containing the same as an active ingredient, particularly a disinfectant and/or an insecticide:

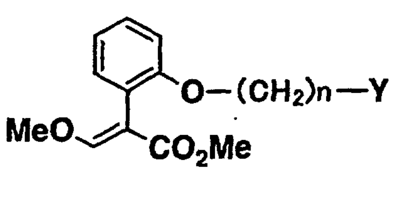

(1)

(wherein n represents an integer from 5 to 12, and Y represents alkyl, haloalkyl, trimethylsilyl or nitryl.)



Printed by Jouve, 75001 PARIS (FR)

## Description

### Field of the Invention

The present invention relates to a novel methyl acetate derivative having disinfectant and insecticidal activity, and a pesticide composition (e.g. a horticultural disinfectant, insecticide etc.) containing the same as an active ingredient.

### Background of the Invention

Both Japanese Patent No. 2,551,555 and Japanese Patent Publication No. 6-80,035 (JP-B-6-80,035) disclose disinfectants belonging to the methoxy acrylate family. For example, as an analogue of the methyl acetate derivative of the present invention, these publications disclose a methyl acrylate derivative having methoxy at position 1 and ortho-substituted phenyl at position 2. In Japanese Patent No. 2,551,555, the ortho substituents of the above phenyl include: $C_{1-4}$ alkyl, $C_{2-5}$ alkenyl or $C_{2-5}$ alkynyl (each of which may be substituted by phenyl, or may be substituted by phenyl having, as substituents, one or more fluorine, chlorine, bromine, $C_{1-4}$ alkyl, $C_{1-4}$ alkoxy or nitro); phenoxy ($C_{1-4}$) alkyl (provided that phenyl may be substituted by one or more halogen, phenyl ring, $C_{1-4}$ alkyl, $C_{1-4}$ alkoxy, nitro, amino or hydroxy); or phenoxy or phenyl ($C_{1-4}$) alkoxy (each of which may be substituted by one or more halogen, methyl, methoxyl, ethyl, ethoxyl or nitro). Furthermore, in Japanese Patent Publication No. 6-80,035, the substituents include phenyl ($C_{1-10}$) alkoxy (provided that an alkylene part thereof may be substituted by alkyl or may have an unsaturated group, and phenyl may be substituted by substituents such as alkyl and haloalkyl.)

Among the above compounds, the methyl acrylate derivatives actually disclosed having ortho-substituted phenyl ether at position 2, include phenoxy or phenyl ($C_{1-10}$) alkoxy wherein the ortho substituent of phenyl is substituted or unsubstituted, and both compounds have a benzene ring at the terminus of the substituents. However, it has been clarified that these compounds do not always provide a sufficient disinfectant activity.

Under these circumstances, in order to find a compound with excellent disinfectant activity which can be used in agricultural industries, the present inventors have synthesized various methyl acetate derivatives, evaluated the biological activities thereof, and performed screenings. Throughout this study, the present inventors have focused particular attention on a methyl acrylate derivative having ortho-substituted phenyl ether at position 2. They have synthesized various compounds having different types of substituents, and compared the biological activities of these compounds with those of conventionally known compounds. As a result, it was surprisingly found that, a specific methyl acetate derivative, an ortho-substituted phenyl ether having a substituted or unsubstituted alkyloxy of carbon number of 5 or more which does not have a benzene ring at the terminus thereof, has more excellent disinfectant activity than the above known compounds, and also has insecticidal activity.

Therefore, the object of the present invention is to provide a novel compound with excellent disinfectant and/or insecticidal activity which can be used in agricultural industries, and a pesticide composition comprising the compound as an active ingredient, particularly a disinfectant and/or an insecticide.

### Summary of the Invention

In one aspect of the present invention, a methyl acetate derivative shown in the following formula (1) is provided:

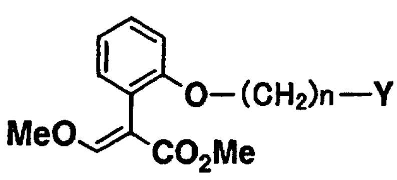

(1)

(wherein n represents an integer from 5 to 12, and Y represents alkyl, haloalkyl, trimethylsilyl or nitryl.)

In one embodiment of the present invention, the above alkyl is linear or branched $C_1$-$C_{12}$ alkyl.

In another embodiment of the present invention, the above haloalkyl is linear or branched $C_1$-$C_4$ alkyl substi-

tuted by one or more halogen atoms.

In a further embodiment of the present invention, in the above formula (1), n represents an integer from 5 to 12, and Y is selected from the group consisting of methyl, ethyl, fluoromethyl, chloromethyl, bromomethyl, iodomethyl, trimethylsilyl and nitryl.

In another aspect of the present invention, a pesticide composition comprising, as an active ingredient, at least one kind of methyl acetate derivative shown in the following formula (1) is provided:

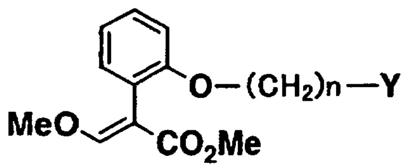

(1)

(wherein n represents an integer from 5 to 12, and Y represents alkyl, haloalkyl, trimethylsilyl, or nitryl.)

In one embodiment of the present invention, the pesticide composition is used as a horticultural disinfectant. More specifically, the pesticide composition is effective in the control of a plant disease selected from the group consisting of *Pyricuraria orizae, Rhizoctonia solani*, *Cochibolus miyabeanus*, powdery mildew, rust, *Phytophthora infestans*, downy mildew, *Venturia inaepualis*, *Alternaria mali*, *Alternaris kikuchiana,* and *Diaporthe citre.*

In another embodiment of the present invention, the pesticide composition is used as an insecticide. More specifically, it is effective in the control of aphids.

In a further embodiment of the present invention, the methyl acetate derivative is a compound shown in the above formula, wherein n represents an integer from 5 to 12, and Y represents linear or branched $C_1$-$C_{12}$ alkyl.

In another embodiment of the present invention, the methyl acetate derivative is a compound shown in the above formula, wherein n represents an integer from 5 to 12, and Y represents linear or branched $C_1$-$C_4$ alkyl substituted by one or more halogen atoms.

In further embodiment of the present invention, the methyl acetate derivative is a compound shown in the above formula, wherein n represents an integer from 5 to 12, and Y is selected from the group consisting of methyl, ethyl, fluoromethyl, chloromethyl, bromomethyl, iodomethyl, trimethylsilyl and nitryl.

## Detailed Description of the Invention

The compounds of the present invention, and control compounds described in the above Japanese Patent No. 2,551,555 including No.153 (methyl (2E) 3-methoxy-2-(2-methoxyphenyl)propa-2-enoate), No.157 (methyl (2E)-2-(2- butoxyphenyl)-3-methoxypropa-2-enoate) and No.159 (methyl (2E)-2-(2-cyclohexyloxyphenyl)-3-methoxypropa-2-enoate) were examined in respect of their ability to control *Pyricuraria orizae*, *Pseudoperonospora cubensis* and *Sphaerotheca fuliginea*,. As a result, it was found that the compounds of the present invention have strikingly superior disinfectant activity in comparison with the above compounds Nos.153, 157 and 159, presented in the above patent, which are analogues of the compounds of the present invention. Furthermore, in respect of the ability to control *Pyricuraria orizae* and *Pseudoperonospora cubensis,* both the compounds of the present invention, and the compounds described in the above Japanese Patent Publication No. 6-80,035 (JP-B-6-80,035) including No.7 (methyl (2E)-3-methoxy-2-[2-(8-phenyloctyloxy)phenyl]propa-2-enoate) and No.120 (methyl (2E)-2-{2-[5-(4-chlorophenyl)pentyloxy]phenyl}-3-methoxypropa-2-enoate) were examined. As a result, it was found that, in this case also, the compounds of the present invention have strikingly superior disinfectant activity in comparison with the above compounds Nos.7 and 120.

Characteristics of the compound of the present invention that were clarified through biological activity tests are, that the compound is effective against one or a plurality of plant pathogenic microbe(s), and that it unexpectedly has an insecticidal effect as well as being effective in controlling plant pathogenic microbes.

The present invention is described in detail below.

In one aspect of the present invention, a methyl acetate derivative shown in the following formula (1) is provided:

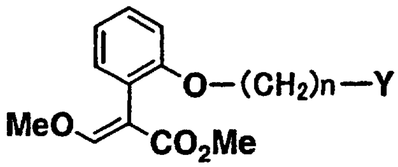

(1)

(wherein n represents an integer from 5 to 12, and Y represents alkyl, haloalkyl, trimethylsilyl or nitryl.)

The methyl acetate derivative of the present invention includes, regarding double bonds thereof, geometric isomers such as syn-form and anti-form, cis form and trans-form; or E-form and Z-form. The present invention includes each of these pure geometric isomers or any mixture thereof. The compound of the present invention has at least pesticidal activity, in particular, disinfectant and/or insecticidal activity, but it can also have other biological activities such as herbicidal activity or plant-controlling activity, depending on the type of compound.

In the above formula (1), n represents an integer from 5 to 12, preferably represents an integer from 5 to 11, and more preferably represents an integer from 5 to 9.

As long as alkyl has disinfectant and/or insecticidal activity substantially equivalent to the compounds of the present invention described in the Examples later, examples of alkyl include, but are not limited to, linear or branched alkyl such as methyl, ethyl, n-propyl, i-propyl, n-butyl, i-butyl, s-butyl, t-butyl, n-pentyl, t-pentyl, hexyl, octyl, dodecyl and the like.

Haloalkyl is alkyl substituted by one or more halogen atoms (e.g. fluorine, chlorine, bromine or iodine). As long as disinfectant and/or insecticidal activity is substantially equivalent to the compounds of the present invention described in the Examples later, examples of haloalkyl include, but are not limited to, bromomethyl, fluoromethyl, chloromethyl, difluoromethyl, dichloromethyl, dibromomethyl, fluorochloromethyl, fluorobromomethyl, chlorobromomethyl, trifluoromethyl, trichloromethyl, tribromomethyl, difluorochloromethyl, difluorobromomethyl, dichlorofluoromethyl, dichlorobromomethyl, dibromofluoromethyl, dibromochloromethyl, 2,2,2-trifluoroethyl, 4,4,4-trifluorobutyl, 4-fluorobutoxy, 4-chlorobutoxy, 4-bromobutoxy and the like.

Apart from the groups listed above, any combination of groups from among the above groups, or selections according to common technical knowledge are also possible.

In one embodiment of the present invention, the methyl acetate derivative of the present invention is a compound, wherein, in the above formula (1), n represents an integer from 5 to 12, and Y represents linear or branched $C_1$-$C_{12}$ alkyl.

In another embodiment of the present invention, the methyl acetate derivative of the present invention is a compound, wherein, in the above formula (1), n represents an integer from 5 to 12, and Y represents linear or branched $C_1$-$C_4$ alkyl substituted by one or more halogen atoms.

In a further embodiment of the present invention, the methyl acetate derivative of the present invention is a compound, wherein, in the above formula (1), n represents an integer from 5 to 12, and Y represents a compound selected from the group consisting of methyl, ethyl, fluoromethyl, chloromethyl, bromomethyl, iodomethyl, trimethylsilyl and nitryl.

Examples of compounds of the present invention are shown in Table 1. In the table, Me, Et, TMS and CN represent methyl, ethyl, trimethylsilyl and nitryl, respectively.

Table 1.

| (List of compounds of the present invention) | | |
|---|---|---|
| Compound No. | n | Y |
| 001 | 5 | Me |
| 002 | 6 | Me |
| 003 | 7 | Me |
| 004 | 8 | Me |
| 005 | 9 | Me |
| 006 | 10 | Me |

Table 1. (continued)

| (List of compounds of the present invention) | | |
|---|---|---|
| Compound No. | n | Y |
| 007 | 11 | Me |
| 008 | 12 | Me |
| 009 | 12 | Et |
| 010 | 5 | $CH_2F$ |
| 011 | 6 | $CH_2F$ |
| 012 | 7 | $CH_2F$ |
| 013 | 8 | $CH_2F$ |
| 014 | 9 | $CH_2F$ |
| 015 | 10 | $CH_2F$ |
| 016 | 11 | $CH_2F$ |
| 017 | 12 | $CH_2F$ |
| 018 | 5 | $CH_2Cl$ |
| 019 | 6 | $CH_2Cl$ |
| 020 | 7 | $CH_2Cl$ |
| 021 | 8 | $CH_2Cl$ |
| 022 | 9 | $CH_2Cl$ |
| 023 | 10 | $CH_2Cl$ |
| 024 | 11 | $CH_2Cl$ |
| 025 | 12 | $CH_2Cl$ |
| 026 | 5 | $CH_2Br$ |
| 027 | 6 | $CH_2Br$ |
| 028 | 7 | $CH_2Br$ |
| 029 | 8 | $CH_2Br$ |
| 030 | 9 | $CH_2Br$ |
| 031 | 10 | $CH_2Br$ |
| 032 | 11 | $CH_2Br$ |
| 033 | 12 | $CH_2Br$ |
| 034 | 5 | $CH_2I$ |
| 035 | 6 | $CH_2I$ |
| 036 | 7 | $CH_2I$ |
| 037 | 8 | $CH_2I$ |
| 038 | 9 | $CH_2I$ |
| 039 | 10 | $CH_2I$ |
| 040 | 11 | $CH_2I$ |
| 041 | 12 | $CH_2I$ |
| 042 | 5 | TMS |
| 043 | 6 | TMS |

Table 1. (continued)

| (List of compounds of the present invention) | | |
|---|---|---|
| Compound No. | n | Y |
| 044 | 7 | TMS |
| 045 | 8 | TMS |
| 046 | 9 | TMS |
| 047 | 10 | TMS |
| 048 | 11 | TMS |
| 049 | 12 | TMS |
| 050 | 5 | CN |
| 051 | 6 | CN |
| 052 | 7 | CN |
| 053 | 8 | CN |
| 054 | 9 | CN |
| 055 | 10 | CN |
| 056 | 11 | CN |
| 057 | 12 | CN |

The compounds of the present invention shown in the above formula (1) can be produced by any applicable methods, and for example, the following method (method A or method B) can be applied.

A method of producing the compound of the present invention according to method A is indicated below:

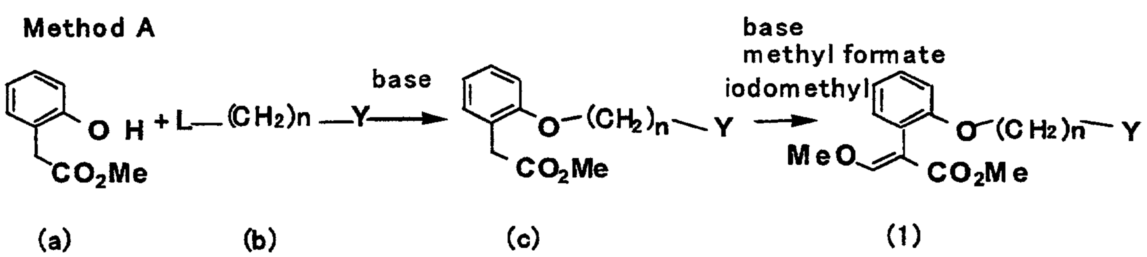


In the above equation, n represents an integer from 5 to 12, Y represents alkyl, haloalkyl, trimethylsilyl or nitryl, and L represents a group capable of being an eliminated group. Examples of alkyl and haloalkyl used herein include the above-stated ones.

Examples of groups that are able to be eliminated group L, include tosyl, mesyl, brosyl, acetyl, halogen and the like, but any group that can be eliminated by nucleophilic attack on the phenoxy of compound (a) can be used.

Compound (a) can be obtained by esterification of commercially available 2-hydroxyphenyl acetate (e.g. nacalai tesque, Inc., Kyoto, Japan) according to standard techniques.

For compound (b), a commercially available halide can be used as is, or it can easily be obtained from a commercially available compound according to standard techniques (e.g. *A New Course of Experimental Chemistry* vol.14 (II), Maruzen, Tokyo, Japan). Examples of production methods of compound (b) include a method of reacting alcohol with phosphorus tribromide at -25°C to at room temperature; a method of reacting alcohol with phosphorus trichloride or phosphorus pentachloride at 0°C to at reflux temperature; and a method of reacting chloride or bromide with silver fluoride in acetonitrile.

Compound (c) can be obtained by reacting compound (a) with compound (b) in an appropriate solvent or without solvent, in the presence or absence of base.

Examples of base used include an inorganic base such as sodium hydroxide, potassium hydroxide and calcium hydroxide; an organic base such as triethylamine and pyridine; a carbonate such as potassium carbonate and

sodium carbonate; and a metallic hydride such as sodium hydride and calcium hydride.

Compound (1) can be obtained, for example, by methoxymethylation of compound (c) according to the description of EP Patent No. 242081.

Reaction temperature can be set to any given temperature within the range from -78°C to 120°C between a freezing point and a boiling point of solvent. As a solvent, though it depends on solubility, any solvent which does not react with the crude material can be used. Examples of solvents include an aliphatic hydrocarbon such as pentane, hexane, heptane and octane; an aromatic hydrocarbon such as benzene, toluene and xylene; an ether such as diethyl ether, tetrahydrofuran, dioxane and dimethoxyethane; halogenated hydrocarbon such as dichloromethane, dichloroethane and chloroform; an alcohol such as methanol, ethanol and isopropyl alcohol; an ester such as methyl acetate and ethyl acetate; a nitryl such as acetonitrile and propionitrile; and dimethylformamide, dimethyl sulfoxide. These solvents can be used alone, or as a mixture, as needed. Reaction time depends on reaction temperature, reaction solvent, reagents and so on, but generally ranges from 0.1 to 48 hours.

The ratio of compounds in each reaction of the above equations is not strictly limited, but for example, the molar ratio of (a) to (b) is generally about 1: about more than 1 (i.e., a excess of (b)), and preferably 0.9 : 1.1.

The separation and purification of each of the compounds generated from reaction mixtures can be performed by known methods such as extraction, recrystallization, chromatography and the like.

The production method of compounds of the present invention by method B is as follows:

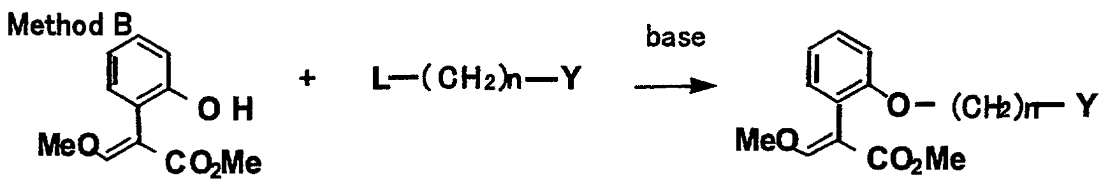


(d) (b) (1)

In the above equation, n represents an integer from 5 to 12, Y represents alkyl, haloalkyl, trimethylsilyl or nitryl, and L represents a group that is able to be an eliminated group. Examples of alkyl and haloalkyl used herein include the above-stated ones.

Examples of groups capable of being an eliminated group include tosyl, mesyl, brosyl, acetyl, halogen and the like, but any group capable of being eliminated due to nucleophilic attack on the phenoxy of compound (a) can be used.

Compound (d) can be obtained by a process of: methylesterificating commercially available 2-hydroxyphenyl acetate (e.g. nacalai tesque, Inc., Kyoto, Japan) according to standard techniques; protecting the hydroxyl group according to standard techniques using THP (tetrahydro-pyranyl), e.g. according to the description in *A New Course of Experimental Chemistry* vol.14 (V), Maruzen, Tokyo, Japan; methoxymethylating the obtained compound e.g. according to the description of EP Patent No. 242081; and removing the protecting group according to standard techniques.

For compound (b), as stated above, commercially available halide can be used as is, or it can easily be obtained from a commercially available compound according to standard techniques (e.g. *A New Course of Experimental Chemistry* vol.14 (I), Maruzen, Tokyo, Japan).

Examples of protecting groups include tetrahydro-pyranyl, tetramethylsilyl, acetyl, benzyl and the like, but are not limited thereto.

Compound (1) can be obtained by reacting compound (d) with compound (b) in an appropriate solvent or without solvent, in the presence or absence of base.

Examples of base used include an inorganic base such as sodium hydroxide, potassium hydroxide and calcium hydroxide; an organic base such as triethylamine and pyridine; a carbonate such as potassium carbonate and sodium carbonate; and a metallic hydride such as sodium hydride and calcium hydride.

Reaction temperature can be set to any given point within the range from -78°C to 120°C between a freezing point and a boiling point of solvent. As a solvent, though it depends on solubility, any solvent which does not react with a crude material can be applied. Examples of solvents include an aliphatic hydrocarbon such as pentane, hexane, heptane and octane; an aromatic hydrocarbon such as benzene, toluene and xylene; an ether such as diethyl ether, tetrahydrofuran, dioxane and dimethoxyethane; halogenated hydrocarbon such as dichloromethane, dichloroethane and chloroform; an alcohol such as methanol, ethanol and isopropyl alcohol; an ester such as methyl acetate and ethyl acetate; a nitrile such as acetonitrile and propionitrile; and dimethylformamide, and dimethyl sulfoxide. These solvents can be used alone, or as a mixture, as needed.

Reaction time depends on reaction temperature, reaction solvent, reagents and so on, but it generally ranges from 0.1 to 48 hours.

The ratio of compounds in each reaction of the above equation is not strictly limited, but for example, the molar ratio of (d) to (b) is generally about 1 : about more than 1 (i.e., an excess of (b)), and preferably 0.9 : 1.1.

The separation and purification of each of the compounds generated from reaction mixtures can be performed by known methods such as an extraction, recrystallization, chromatography and the like.

Furthermore, the present invention relates to a pesticide composition (e.g. a horticultural disinfectant, insecticide, herbicide, plant regulator etc.) containing, as an active ingredient, at least one compound from among the compounds shown in the above formula (1), preferably a disinfectant and/or an insecticide.

The methyl acetate derivative shown in formula (1) of the present invention is useful as an active ingredient of a horticultural disinfectant, and it has an extremely high efficacy in the control of plant diseases including: *Pyricuraria orizae*, *Rhizoctonia solani*, *Cochibolus miyabeanus*, *Eysiphe graminis*, *Puccinia coronato*, *Phytophthora infestans* and others; downy mildew against various plants, i.e., *Pseudoperonospora cubensis* and *Plasmopara viticola*; and other plant diseases such as *Venturia inaepualis, Alternaria mali*, *Alternaris kikuchiana* and *Diaporthe citre.*

The methyl acetate derivative of the present invention can be used as an insecticide. In the embodiment of the present invention, the above derivative has insecticidal activity against aphids such as green peach aphids.

Where the compound of the present invention is used as a disinfectant and/or an insecticide, this compound may be used as is. However, in order to effectively disperse the active ingredient thereof to a target location, this compound may be mixed with a carrier, diluent, additive, adjuvant and the like by known means prior to use, and can be prepared as a general formulation for a pesticide, e.g. powders, granules, wettable powders, emulsifiable concentrates, suspension concentrates and the like. Furthermore, this compound may be also used by mixing with or using in combination with other pesticides, e.g. a disinfectnant, insecticide, acaricide, herbicide, plant growth-regulating agent, fertilizer, ameliorant and the like.

The horticultural disinfectant and insecticide of the present invention may can used mixed with other agent, e.g. a disinfectant, insecticide, acaricide etc, which do not contain active ingredients inhibiting the disinfectant or insecticidal effect of the present invention. It is expected that the mixed use of horticultural disinfectant and insecticide of the present invention with other disinfectants, insecticides and acaricides does not only result in reduction of the amount of pesticide used, and labor-saving, but also the expansion of disinfectant and insecticidal spectrum, and an extremely high effect due to the synergy of both agents.

Carriers or diluents used for pharmaceutical manufacturing include solid or liquid carriers generally used for pesticide formulations.

Examples of solid carriers include, but are not limited to: inorganic substances such as kaolinite, montmorillonite, illite, polygloskite, pioflite, attapulgite, vermiculite, sepiolite, kaolinite, bentonite, mica, talc, gyps, calcium carbonate, dolomite, white carbon, diatomaceous earth, magnesium calcium, apatite, zeolite, silica, synthetic calcium silicate and others; inorganic salts of organic acids such as magnesium stearate, calcium stearate and calcium citrate; plant organic substances such as soy flour, tobacco flour, walnut flour, wheat flour, wood flour, starch and crystalline cellulose; synthetic or natural high molecular substances such as chromane resin, petroleum resin, alkyd resin, polyvinyl chloride, polyalkylenglycol, ketone resin, ester gum, corbal gum and gum dammar; wax groups such as carnauba wax and yellow wax; or urea.

Examples of liquid carriers include, but are not limited to: paraffin or naphthene hydrocarbon such as kerosine, mineral oil, spindle oil and white oil; aromatic hydrocarbon such as xylene, ethylbenzene, cumene and methylnaphthalene; hydrocarbon chloride such as trichloroethylene and chlorobenzene; ether such as dioxane and tetrahydrofuran; ketone such as acetone, methylethylketone, diisopropyl keton, cyclohexanone, acetophenone and isophorone; ester such as ethyl acetate, amyl acetate, ethylene glycol acetate, diethylene glycol acetate, dibutyl maleate and diethyl succinate; alcohol such as methanol, hexanol, ethylene glycol, diethylene glycol and cyclohexanol; ether such as ethylene glycol diethylether, diethylene glycol and dibutyl ether; polar solvents such as dimethylformamide, dimethyl sulfoxide and water; or silicone oil.

In addition, one or more than one kind of surfactant or other adjuvant (e.g. a spreader, emulsifier, moistening agent, dispersant, anchorage, disintegrator and the like) can be applied to the compound of the present invention, for the purpose of: control regarding emulsification, dispersion, moistening, spreading, binding, sticking and disintegrating; stabilization of active ingredients; improvement of flowability; rust inhibition; antifreezing; and the like.

Examples of nonionic surfactants used include, but are not limited to: compounds obtained by the polymerization of higher alcohol such as lauryl alcohol, stearyl alcohol and oleyl alcohol to ethylene oxide; compounds obtained by the polymerization of higher fatty acid such as palmitin acid, stearyl acid and oleyl acid to ethylene oxide; polyalcohol higher fatty acid ester such as sorbitan and a compound obtained by the polymerization of the above compound to ethylene oxide; and silicone surfactants.

Examples of anionic surfactants used include, but are not limited to: alkyl ester sulfate such as sodium lauryl sulfate and oleyl alcohol sulfate ester amine; alkyl sulfonate such as dioctyl ester sodium sulfosuccinate; aryl sulfonate

of sodium aryl sulfonate such as sodium lignosulfonate and sodium dodecylbenzenesulfonate.

Furthermore, high molecular compounds such as casein, gelatin, albumin, glue, sodium alginate, CMC, methyl cellulose, xanthan gum and polyvinyl alcohol, and other adjuvants can be used in combination with the disinfectant and insecticide of the present invention to improve the properties of the formulation and enhance disinfectant effect thereof.

The amount of active ingredient of the present compound in various formulations obtained as above depends on those formulations, but in general, it makes up 0.01 to 90 parts, and preferably 1 to 60 parts per 100 parts of formulation. Unless otherwise specified, "part" used herein means part by weight.

Where the compound of the present invention is wettable powder, an active ingredient thereof generally makes up 1 to 90 parts, preferably 5 to 75 parts per 100 parts of formulation, the remainder consists of a solid carrier and a dispersing-moistening agent, and as necessary, a protective colloid and an antifoaming agent are also added thereto. A dispersing-moistening agent, protective colloid and antifoaming agent used herein are ones commonly used in the art.

Where the compound of the present invention is in the form of granules, an active ingredient thereof generally makes up 0.01 to 40 parts, preferably 0.1 to 20 parts per 100 parts of formulation, the remainder consists of a solid carrier, a surfactant and the like. The active ingredient is homogeneously mixed with a solid carrier, or homogeneously adsorbed or fixed onto the surface of a solid carrier. The diameter of a granule is normally 0.1 to 2.0mm.

Where the compound of the present invention is an emulsifiable concentrate, an active ingredient thereof generally makes up 1 to 60 parts, preferably 5 to 40 parts per 100 parts of formulation which contains a suitable amount of emulsifier is added thereto, and the remainder consists of a liquid carrier, and as necessary, a spreader, a deodorant and the like are also added thereto. The spreader and deodorant used herein are the ones which are commonly used in the art.

Where the compound of the present invention is a suspension concentrate, an active ingredient thereof generally makes up 1 to 80 parts, preferably 5 to 50 parts per 100 parts of formulation which contains a suitable amount of dispersing-moistening agent, and the remainder consists of water, and as necessary, a protective colloid, an antiseptic and an antifoaming agent are also added thereto.

Where the compound of the present invention is dust, an active ingredient thereof generally makes up 0.01 to 10 parts, preferably 0.1 to 5 parts per 100 parts of formulation which contains a suitable amount of dispersing moistening agent, and the rest consists of water, and as necessary, a protective colloid, an antiseptic and an antifoaming agent are also added thereto.

The methyl acetate of the present invention can be used as a compound shown in the above formula (1), or as any of the formulations described above.

Depending on target crop, usage, type of formulation, the whole dosage used and the like, the dosage and concentration of the compound of the present invention differ, but when the compound is applied to foliage for example, the concentration of active ingredient is 0.1 to 1,000ppm, preferably 0.5 to 500ppm. When the compound is applied to soil, the concentration is 1 to 10,000g/10are, preferably 10 to 2,000g/10are.

## Example

The production examples of the compound of the present invention by methods A and B, the examples of formulations and the biological activity test examples of the above compound are further described in the following examples. The example is provided for illustrative purposes only, and is not intended to limit the scope of the invention.

### Production example 1.

### Synthesis of methyl(2E)-3-methoxy-2-(octyloxyghenyl)propa-2-enoate (Compound No. 003) by Method A

44.34g of 2-(2-hydroxyphenyl) acetate (which can be obtained from e.g. nacalai tesque, Inc., Kyoto, Japan) was dissolved in 150ml of methanol, followed by addition of 0.3ml of concentrated sulfuric acid thereto, and the mixture was then stirred overnight at room temperature. After methanol was removed under reduced pressure, saturated sodium bicarbonate solution and ethyl acetate were added thereto, extracted with ethyl acetate (3 times), and dehydrated with sodium sulfate. Recrystallization was performed from hexane-ethyl acetate, and a white crystal, 2-(2-hydroxyphenyl) acetate (46.82g, yield: 97%) was obtained.

Thereafter, 4.98g of methyl 2-(2-hydroxyphenyl) acetate and 6.37g of octyl bromide were dissolved in 50ml of DMF, followed by addition of 6.90g of potassium carbonate thereto, and then the mixture was stirred overnight at room temperature. Then, 50ml of saturated brine solution was added to the mixture, and the mixture was extracted with ethyl acetate (3 times), washed with dilute hydrochloric acid, saturated sodium bicarbonate solution and saturated brine solution, and dehydrated with magnesium sulfate. After solvents were removed under reduced pressure, 11.25g of liver-colored liquid was obtained. The obtained liquid was purified by a silica gel column chromatography (hexane :

ethyl acetate = 80 : 1), and an achromatic oily product, methyl 2-(2-octyloxyphenyl) acetate (3.50g, yield: 42%) was obtained.

Subsequently, 300mg of 60% sodium hydride was suspended in 3ml of DMF, and 6ml of DMF containing 830mg of methyl-2-(2-octyloxyphenyl) acetate was added drop-wise thereto at room temperature over about 5 minutes. After stirring at room temperature for 1 hour, 1ml of methyl formate was added drop-wise to the mixture at room temperature, followed by stirring for 8 hours. Then, 1ml of iodomethyl was added thereto and the mixture was stirred at room temperature for 1 hour. Thereafter, 20ml of saturated brine solution was added to the reacted mixture, which was then extracted with methyl acetate (3 times), washed with dilute hydrochloric acid, saturated sodium bicarbonate solution and saturated brine solution, and dehydrated with magnesium sulfate. After solvents were removed under reduced pressure, 1.18g of pale yellow oily product was obtained. The obtained product was purified by silica gel column chromatography (hexane : ethyl acetate = 10 : 1), and an achromatic oily product, methyl(2E)-3-methoxy-2-(octyloxyphenyl) propa-2-enoate (319mg, yield: 33%) was obtained. $^1$H NMR ($CDCl_3$): δ (ppm) 7.48 (s, 1H), 7.28-6.97 (m, 4H), 3.93 (t, 2H), 3.81 (s, 3H), 3.69 (s, 3H), 1.70 (m, 2H), 1.43-1.23 (m, 10H), 0.80 (t, 3H).

Production example 2.

Synthesis of methyl(2E)-2-[2-(6-bromohexyloxy)phenyl]-3-methoxypropa-2-enoate (Compound No. 026) by Method B

Methyl-2-(2-methylphenyl)acetate (which can be obtained from e.g. nacalai tesque, Inc., Kyoto, Japan), 4.31g, was dissolved in 15ml of 3,4-dihydropyran (which can be obtained from e.g. Aldrich, USA), and while cooling with ice, a small amount of camphorsulfonic acid was added thereto followed by stirring for 30 minutes. Saturated sodium bicarbonate solution, 20ml, was added to the reaction mixture, and the mixture was extracted with ethyl acetate 3 times, washed with saturated brine solution and dehydrated with magnesium sulfate. After solvents were removed under reduced pressure, 7.60g of pale yellow oily product was obtained. The obtained product was purified by silica gel column chromatography (hexane : ethyl acetate = 20 : 1), and an achromatic oily product, methyl 2-(2-perhydro-2H-pyran-2-iloxyphenyl)acetate (6.06g, yield: 93%) was obtained.

After that, 2.28g of 60% sodium hydride was suspended in 40ml of DMF, and 40ml of DMF containing 5.68g of methyl 2-(2-perhydro-2H-pyrane-2-iloxyphenyl) acetate and 5.7g of methyl formate was dropped thereto at room temperature over about 5 minutes, followed by stirring for 8 hours. Then, 2ml of iodomethyl was added thereto and the mixture was stirred at room temperature for 1 hours. After that, 40ml of saturated brine solution was added to the reacted mixture, and the mixture was extracted with ethyl acetate (3 times), washed with saturated sodium bicarbonate solution and saturated brine solution, and dehydrated with magnesium sulfate. After solvents were removed under a reduced pressure, 11.00g of pale yellow oily product was obtained. The obtained product was dissolved in 60ml of methanol, and 40ml of IN hydrochloric acid was added thereto, followed by stirring at room temperature for 30 minutes. Then, 100ml of sodium bicarbonate solution was added to the reacted mixture, and the mixture was extracted with ethyl acetate 3 times, washed with dilute hydrochloric acid, saturated sodium bicarbonate solution and saturated brine solution, and dehydrated with magnesium sulfate. After solvents were removed under a reduced pressure, 4.42g of pale yellow oily product was obtained. The obtained product was purified by silica gel column chromatography (hexane : ethyl acetate = 5 : 1), and an achromatic crystal, methyl(2E)-2-(2-hydroxyphenyl)-3-methoxypropa-2-enoate (1.90g, yield: 40%) was obtained.

Subsequently, 624mg of methyl(2E)-2-(2-hydroxyphenyl)-3-methoxypropa-2-enoate and 672mg of 1,6-dibromohexane (which can be obtained from e.g. Aldrich, USA) were dissolved in 10ml of DMF, followed by addition of 552mg of potassium carbonate thereto, and the mixture was stirred overnight at room temperature. Then, 10ml of saturated brine solution was added to the reacted mixture, and the mixture was extracted with ethyl acetate 3 times, washed with dilute hydrochloric acid, saturated sodium bicarbonate solution and saturated brine solution, and dehydrated with magnesium sulfate. After solvents were removed under a reduced pressure, 940mg of liver-colored oily product was obtained. The obtained product was purified by silica gel column chromatography (hexane : ethyl acetate = 40 : 1), and an achromatic oily product, methyl(2E)-2-[2-(6-bromohexyloxy)phenyl]-3-methoxypropa-2-enoate (189mg, yield: 17%) was obtained. $^1$H NMR ($CDCl_3$): δ (ppm) 7.39 (s, 1H), 7.17 (m, 1H), 7.10 (dd, 1H), 6.86 (dd, 1H), 6.80 (d, 1H), 3.85 (t, 2H), 3.71 (s, 3H), 3.59 (s, 3H), 3.28 (t, 2H), 1.80 (m, 2H), 1.64 (m, 2H), 1.45-1.30 (m, 4H).

Production example 3.

Synthesis of compounds shown as compound Nos. 001, 002, 005, 007, 010, 028 and 042

The above-identified compounds (see Table 1) used in the examples described below were synthesized by Method B, following the processes described in production example 2. Table 2 shows the results of NMR spectrum analysis.

Table 2.

| Compound No. | $^1$H NMR ($CDCl_3$): δ (ppm) |
|---|---|
| 001 | 7.45(s,1H),7.35(m,1H),7.20(m,1H),6.90(m,2H),3.90(t,2H),3.80(s ,3H),3.65(s,3H),1.75(m,2H), 1.45-1.20(m,8H),0.95(t,3H) |
| 002 | 7.40(s,1H),7.30(m,1H),7.22(m,1H),6.95(m,2H),3.92(t,2H),3.83(s ,3H),3.62(s,3H),1.70(m,2H), 1.45-1.10(m,10H),0.92(t,3H) |
| 005 | 7.40(s,1H),7.32(m,1H),7.22(m,1H),6.92(m,2H),3.90(t,2H),3.82(s ,3H),3.65(s,3H),1.72(m,2H), 1.48-1.20(m,16H),0.90(t,3H) |
| 007 | 7.40(s,1H),7.30(m,1H),7.25(m,1H),6.92(m,2H),3.95(t,2H),3.80(s ,3H),3.60(s,3H),1.71 (m,2H), 1.50-1.18(m,20H),0.90(t,3H) |
| 010 | 7.65 (s, 1H), 7.22 (m, 1H), 7.09 (dd, 1H), 6.93 (dd, 1H), 6.38 (d, 1H), 4.43 (dt, 2H), 3.93 (t, 2H), 3.76 (s, 3H), 1.76-1.62 (m, 4H), 1.48-1.40 (m, 4H) |
| 028 | 7.48 (s, 1H), 7.29-7.24 (m, 1H), 7.20-7.16 (m, 1H), 6.96-6.87 (m, 2H), 3.93 (t, 2H), 3.82 (s, 3H), 3.68 (s, 3H), 1.90-1.30 (m, 12H) |
| 042 | 7.28 (s, 1H), 7.25 (m, 1H), 7.19 (m, 1H), 6.91 (m, 1H), 6.87 (m, 1H), 3.97 (t, 2H), 3.70 (s, 3H), 3.65 (s, 3H), 1.78 (m, 2H), 1.56-0.93 (m, 6H), 0.00 (s, 9H) (?) |

Some examples of formulations containing the compounds of the present invention synthesized above are shown below.

Formulation example 1. (wettable powder)

| | |
|---|---|
| Compound No. 003 | 25 parts |
| Diatomaceous earth | 65 parts |
| Calcium lignosulfonate | 5 parts |
| Formaldehyde condensation naphthalenesulfonate | 5 parts |

After mixing the above compounds, the mixture was crashed with a hammer mill to obtain wettable powders.

Formulation example 2. (suspension concentrate)

| | |
|---|---|
| Compound No. 003 | 30 parts |
| Di-2-ethylhexyl ester sodium sulfosuccinate | 2 parts |
| Polyoxyethylene nonylphenyl ether | 2 parts |
| Silicone resin | 1 part |
| Propylene glycol | 5 parts |
| Water | 60 parts |

A suspension concentrate was obtained by uniformly crashing and mixing the above compounds with a ball mill.

Formulation example 3. (granule)

| | |
|---|---|
| Compound No. 003 | 20 parts |
| Diatomaceous earth | 68 parts |

(continued)

| | |
|---|---|
| Calcium lignosulfonate | 5 parts |
| Formaldehyde condensation naphthalenesulfonate | 5 parts |
| Polyvinyl alcohol | 2 parts |

Granules were obtained by kneading the above compounds with a kneader followed by extrusion of the obtained mixture.

Formulation example 4. (emulsifiable concentrate)

| | |
|---|---|
| Compound No. 003 | 10 parts |
| Xylene | 50 parts |
| Polyoxyethylene snonylphenyl ether | 5 parts |
| Polyoxyethylene styrylphenyl ether | 5 parts |
| Dimethyl sulfoxide | 30 parts |

An emulsifiable concentrate was obtained by uniformly mixing and dissolving the above compounds with a homomixer.

The efficacy of the above-synthesized compounds of the present invention in the control of various plant diseases is demonstrated in the following test examples.

Test example 1. Test of control activity against *Pyricuraria orizae*

8 rice plants (variety: Jikkoku, home seed-raising) cultivated in each of 4 plastic pots containing compost were used as test plants.

The wettable powder containing each of the test compounds prepared by a method described in Formulation example 1 was diluted with water to prepare two agent solutions, whose final concentrations are 100ppm and 10ppm. Using a spray gun, 10ml of each of the prepared agent solutions was dispersed to the 3 pots. After air-drying the agent solutions, a conidium suspension of *Pyricuraria orizae* ($1 \times 10^6$/ml), which was prepared by previously culturing in an oat meal agar medium (oat meal 5%, sucrose 1%, agar 1%) at 28°C for 8 days, was uniformly sprayed to inoculate, then kept at rest overnight at 24°C in a moist chamber. After that, the plants were transferred into an artificial climate chamber whose temperature was set to 18°C by night and to 25°C by day to allow the disease to develop. At 7 days after inoculation, the number of lesion was counted, and an average lesion number per treated rice plant was determined. Moreover, the average lesion number was determined also for a non-treated rice plant, to which the agent solution was not dispersed, but the conidium suspension of *Pyricuraria orizae* was inoculated in the same way as stated above so as to allow the disease to develop. Then, according to the following formula, a disease-control value of each compound was calculated.

As control compounds, compound Nos. 153, 157 and 159 (the compound names of each of which was stated above), which were suggested in Japanese Patent No. 2,551,555, were used.

Disease-control value = (1-the average lesion number of a treated plant/the average lesion number of a non-treated plant) × 100

As a result of this test, the compounds showing a good activity are shown in Table 3. No phytotoxicity was detected.

Table 3.

| Test of control activity against *Pyricuraria orizae* | | |
|---|---|---|
| Compound No. | 100 ppm | 10 ppm |
| 001 | 100 | 90 |
| 003 | 100 | 90 |
| 005 | 100 | 90 |
| 007 | 100 | 80 |

Table 3. (continued)

| Test of control activity against *Pyricuraria orizae* | | |
|---|---|---|
| Compound No. | 100 ppm | 10 ppm |
| 042 | 100 | 70 |
| No. 153 | 30 | 0 |
| No. 157 | 50 | 0 |
| No. 159 | 40 | 0 |
| non-treated | 0 | 0 |

Test example 2. Test of control activity against *Pseudoperonospora cubensis*

Cucumber plants in the cotyledon period (variety: Suyo, home seed-raising) cultivated in plastic pots containing compost were used as test plants.

The wettable powder containing each of the test compounds prepared by a method described in Formulation example 1 was diluted with water to prepare two agent solutions, whose final concentrations are 100ppm and 10ppm. Using a spray gun, 5ml of each of the prepared agent solutions was dispersed to the 3 pots. After air-drying the agent solutions, a conidium suspension of *Pseudoperonospora cubensis* ($1 \times 10^6$/ml), which was prepared after collection from a cucumber leaf previously infected with *Pseudoperonospora cubensis,* was uniformly sprayed to inoculate, then kept at rest overnight at 21°C in a moist chamber. After that, the plants were transferred into an artificial climate chamber, whose temperature was set to 18°C by night and to 22°C by day to allow the disease to develop. At 7 days after inoculation, the area where lesions appeared was measured. Then, applying the following exponents, the incidence rate of each of the treated plants was calculated. Moreover, the incidence rate was calculated also for a non-treated cucumber plant, to which agent solution was not dispersed, but the conidium suspension of *Pseudoperonospora cubensis* was inoculated in the same way as stated above so as to allow the disease to develop. Then, according to the following exponents and formula, a disease-control value of each compound was calculated. Furthermore, as control compounds, compound Nos. 153 and 157 suggested in Japanese Patent No. 2,551,555 were used.
Exponents

0: no lesion
1: a number of lesions
2: damaged area makes up less than one quarter of an entire leaf
3: damaged area makes up more than one quarter to less than one half of an entire leaf
4: damaged area makes up more than one half to less than three quarter of an entire leaf
5: damaged area makes up more than three quarter of an entire leaf

$$\text{Incidence rate} = \Sigma\,(\text{exponent} \times \text{applicable plant number})/(\text{searched plant number} \times 5) \times 100$$

$$\text{Disease-control value} = (1 - \text{the incident rate of a treated plant/the incident rate of a non-treated plant}) \times 100$$

As a result of this test, the compounds showing good activity are shown in Table 4. No phytotoxicity was detected..

Table 4.

| Test of control activity against *Pseudoperonospora cubensis* | | |
|---|---|---|
| Compound No. | 100 ppm | 10 ppm |
| 001 | 100 | 70 |

Table 4. (continued)

| Test of control activity against *Pseudoperonospora cubensis* | | |
|---|---|---|
| Compound No. | 100 ppm | 10 ppm |
| 003 | 100 | 90 |
| 042 | 90 | 60 |
| No. 153 | 30 | 10 |
| No. 157 | 20 | 10 |
| non-treated | 0 | 0 |

Test example 3. Test of disease-control activity against *Sphaerotheca fuliginea*

Cucumber plants in the cotyledon period (variety: Suyo) cultivated in plastic pots containing compost were used as test plants.

The wettable powder containing each of the test compounds prepared by a method described in Formulation example 1 was diluted with water to prepare two agent solutions, whose final concentrations are 100ppm and 10ppm. Using a spray gun, 5ml of each of the prepared agent solutions was dispersed to the 3 pots. After air-drying the agent solutions, *Sphaerotheca fuliginea* was inoculated to the test plants, using pumpkin leaves previously infected with powdery mildew. After that, the cucumber plants were transferred into an artificial climate chamber whose temperature was set to 18°C by night and to 25°C by day to develop the disease. At 7 days after inoculation, the area where lesions appeared was measured. Then, applying the following exponents, the incidence rate of each of the treated plants was calculated. Moreover, the incidence rate was calculated also for a non-treated cucumber plant, to which agent solution was not dispersed, but the conidium suspension of *Sphaerotheca fuliginea* was inoculated in the same way as stated above so as to allow the disease to develop. Then, the incidence rate of the non-treated plant was determined, and finally the disease-control value of each of the plants was calculated according to the following exponents and formula. Furthermore, as control compounds, compound Nos. 153 and 157 suggested in Japanese Patent No. 2,551,555 were used.
Exponents

0: no lesion
1: a number of lesions
2: damaged area makes up less than one quarter of an entire leaf
3: damaged area makes up more than one quarter to less than one half of an entire leaf
4: damaged area makes up more than one half to less than three quarter of an entire leaf
5: damaged area makes up more than three quarter of an entire leaf

$$\text{Incidence rate} = \Sigma(\text{exponent} \times \text{applicable plant number})/(\text{searched plant number} \times 5) \times 100$$

$$\text{disease-control value} = (1 - \text{the incident rate of a treated plant/the incident rate of a non-treated plant}) \times 100$$

As a result of this test, the compounds showing good activity are shown in Table 5. No phytotoxicity was detected.

Table 5.

| Test of control activity against *Sphaerotheca fuligineas* | | |
|---|---|---|
| Compound No. | 100 ppm | 10 ppm |
| 002 | 100 | 60 |

Table 5. (continued)

| Test of control activity against *Sphaerotheca fuligineas* | | |
|---|---|---|
| Compound No. | 100 ppm | 10 ppm |
| 003 | 100 | 60 |
| 005 | 100 | 80 |
| 042 | 100 | 100 |
| No. 153 | 0 | 0 |
| No. 157 | 0 | 0 |
| non-treated | 0 | 0 |

Test example 4. Test of control activity against *Pyricuraria orizae*

8 rice plants (variety: Jikkoku) cultivated in each of 4 plastic pots containing compost were used as test plants.

The wettable powder containing each of the test compounds prepared by a method described in Formulation example 1 was diluted with water to prepare two agent solutions, whose final concentrations were 100ppm and 10ppm. Using a spray gun, 10ml of each of the prepared agent solutions was dispersed to the 3 pots. After air-drying the agent solutions, a conidium suspension of *Pyricuraria orizae* ($1 \times 10^6$/ml), which was prepared after previously culturing in an oat meal agar medium (oat meal 5%, sucrose 1%, agar 1%) at 28°C for 8 days, was uniformly sprayed to inoculate, then kept at rest overnight at 24°C in a moist chamber. After that, the plants were transferred into an artificial climate chamber whose temperature was set to 18°C by night and to 25°C by day to allow the disease to develop. At 7 days after inoculation, the number of lesions was counted, and the average lesion number per a treated rice plant was determined. Moreover, the average lesion number was determined also for a non-treated rice plant, to which the agent solution was not dispersed, but the conidium suspension of *Pyricuraria orizae* was inoculated in the same way as stated above in order to allow the disease to develop. Then, according to the following formula and standards, the disease-control value of each compound was calculated and effectiveness determined. As control compounds, compound Nos. 7 and 120 (the compound names of each of which were stated above), which were described in Japanese Patent Publication No. 6-80,035, were used.

Disease-control value = (1 — the average lesion number of a treated plant/the average lesion number of a non-treated plant) × 100

| | |
|---|---|
| Effect A: control value | 100 to 90% |
| Effect B: control value | 89 to 70% |
| Effect C: control value | 69 to 50% |
| Effect D: control value | 49 to 0% |

As results for this test, the compounds showing good activity are shown in Table 6. No phytotoxicity was detected..

Table 6.

| Test of control activity against *Pyricuraria orizae* | | |
|---|---|---|
| Compound No. | 100 ppm | 10 ppm |
| 003 | A | A |
| 026 | A | A |
| 028 | A | A |
| No. 7 | C | D |
| No. 120 | C | D |

Table 6. (continued)

| Test of control activity against *Pyricuraria orizae* | | |
|---|---|---|
| Compound No. | 100 ppm | 10 ppm |
| non-treated | D | D |

Test example 5. Test of control activity against *Sphaerotheca fuliginea*

Cucumber plants in the cotyledon period (variety: Suyo) cultivated in plastic pots containing compost were used as test plants. The wettable powder containing each of the test compounds prepared by a method described in Formulation example 1 was diluted with water to prepare two agent solutions, whose final concentrations are 100ppm and 10ppm. Using a spray gun, 5ml of each of the prepared agent solutions was dispersed to the 3 pots. After air-drying the agent solutions, *Sphaerotheca fuliginea* was inoculated to the test plants, using pumpkin leaves previously infected with powdery mildew. After that, the plants were transferred into an artificial climate chamber, whose temperature was set to 18°C by night and to 25°C by day to allow the disease to develop. At 7 days after inoculation, the area where lesions appeared was measured, and applying the following exponents, the incidence rate of each of the treated plants was calculated. Moreover, the incidence rate was calculated also for a non-treated cucumber plant, to which agent solution was not dispersed, but the conidium suspension of *Sphaerotheca fuliginea* was inoculated in the same way as stated above so as to allow the disease to develop. Then, the incidence rate of the non-treated plant was determined, and finally a disease-control value for each of the plants was calculated according to the following formula and exponents. Furthermore, as control compounds, compound Nos. 7 and 120 described in Japanese Patent Publication No. 6-80,035, were used.
Exponents

0: no lesion
1: a number of lesions
2: damaged area makes up less than one quarter of an entire leaf
3: damaged area makes up more than one quarter to less than one half of an entire leaf
4: damaged area makes up more than one half to less than three quarter of an entire leaf
5: damaged area makes up more than three quarter of an entire leaf

$$\text{Incidence rate} = \Sigma\,(\text{exponent} \times \text{applicable plant number}) / (\text{searched plant number} \times 5) \times 100$$

$$\text{Disease-control value} = (1 - \text{the incident rate of a treated plant / the incident rate of a non-treated plant}) \times 100$$

| | |
|---|---|
| Effect A: disease-control value | 100 to 90% |
| Effect B: disease-control value | 89 to 70% |
| Effect C: disease-control value | 69 to 50% |
| Effect D: disease-control value | 49 to 0% |

As a result of this test, the compounds showing a good activity are shown in Table 7. No phytotoxicity was detected..

Table 7.

| Test of control activity against *Sphaerotheca fuliginea* | | |
|---|---|---|
| Compound No. | 100 ppm | 10 ppm |
| 003 | A | A |

Table 7. (continued)

| Test of control activity against *Sphaerotheca fuliginea* | | |
|---|---|---|
| Compound No. | 100 ppm | 10 ppm |
| 026 | A | A |
| 028 | A | A |
| No. 7 | C | D |
| No. 120 | C | D |
| non-treated | D | D |

Test example 6. Test of insecticidal activity against green peach aphid

Each of the test compounds, which were diluted to an established concentration, was dispersed with a spray gun to a cabbage leaf disk, with a diameter of 5 cm, placed in a plastic cup, followed by air-drying. Ten green peach aphid larvae of the first instar were left in the cup, and after covering the cup with a lid, these were fed at 25° in a low temperature chamber. At 3 days after the above treatment, the viability of the aphid larvae was determined, and the death rate was calculated according to the following formula and criteria and the effects of the compounds determined.

Death rate (%) = (total number of aphid larvae left — aphid larvae number surviving) /

total number of aphid larvae left × 100

| | |
|---|---|
| Effect A: death rate | 100 to 90% |
| Effect B: death rate | 89 to 80% |
| Effect C: death rate | 79 to 60% |
| Effect D: death rate | 59 to 0% |

As results for this test, compounds showing high insecticidal activity are shown in Table 8.

Table 8.

| Test of insecticidal activity against green peach aphid | |
|---|---|
| Compound No. | Effect |
| 002 | A |
| 003 | A |
| 010 | A |
| non-treated | D |

Industrial Applicability

The methyl acetate derivative of the present invention is useful as a horticultural disinfectant and/or an insecticide, in that it has an extremely high disinfectant activity in comparison with conventional compounds having a structural similar to the present derivative, and that it has excellent insecticidal activity which has not been disclosed yet.

All publications cited in the present application are hereby incorporated by reference herein. While there have been described what are presently believed to be the preferred embodiments of the invention, those skilled in the art will realize that changes and modifications may be made without departing from the spirit of the invention. It is intended to claim all such changes and modifications as fall within the true scope of the invention.

## Claims

**1.** A methyl acetate derivative shown in the following formula (1):

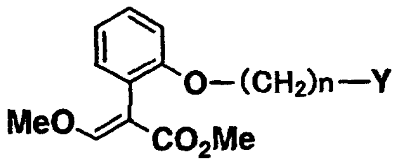

(1)

(wherein n represents an integer from 5 to 12, and Y represents alkyl, haloalkyl, trimethylsilyl or nitryl.)

**2.** The methyl acetate derivative according to claim 1, wherein said alkyl is linear or branched $C_1$-$C_{12}$ alkyl.

**3.** The methyl acetate derivative according to claim 1, wherein said haloalkyl is linear or branched $C_1$-$C_4$ alkyl substituted with one or more halogen atoms.

**4.** The methyl acetate derivative according to claim 1, wherein, in said formula (1), n represents an integer from 5 to 12, and Y is selected from the group consisting of methyl, ethyl, fluoromethyl, chloromethyl, bromomethyl, iodomethyl, trimethylsilyl and nitryl.

**5.** A pesticide composition comprising, as an active ingredient, at least one kind of methyl acetate derivative shown in the following formula (1):

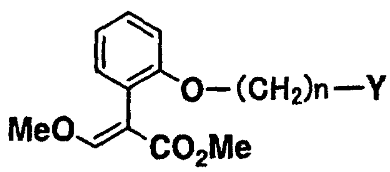

(1)

(wherein n represents an integer from 5 to 12, and Y represents alkyl, haloalkyl, trimethylsilyl or nitryl.)

**6.** The pesticide composition according to claim 5, which is used as a horticultural disinfectant.

**7.** The pesticide composition according to claim 6, which is effective in controlling a plant disease selected from the group consisting of *Pyricuraria orizae, Rhizoctonia solani*, *Cochibolus miyabeanus,* powdery mildew, rust, *Phytophthora infestans*, downy mildew, *Venturia inaepualis*, *Alternaria mali*, *Alternaris kikuchiana*, and *Diaporthe citre.*

**8.** The pesticide composition according to claim 5, which is used as an insecticide.

**9.** The pesticide composition according to claim 8, which is effective in controlling aphids.

**10.** The pesticide composition according to claim 5, wherein said methyl acetate derivative is a compound according to said formula (1), wherein n represents an integer from 5 to 12, and Y is linear or branched $C_1$-$C_{12}$ alkyl.

**11.** The pesticide composition according to claim 5, wherein said methyl acetate derivative is a compound according to said formula (1), wherein n represents an integer from 5 to 12, and Y is linear or branched $C_1$-$C_4$ alkyl substituted by one or more halogen atoms.

**12.** The pesticide composition according to claim 5, wherein said methyl acetate derivative is a compound according to said formula (1), wherein n represents an integer from 5 to 12, and Y is selected from the group consisting of methyl, ethyl, fluoromethyl, chloromethyl, bromomethyl, iodomethyl, trimethylsilyl and nitryl.

## INTERNATIONAL SEARCH REPORT

International application No.
PCT/JP99/05647

A. CLASSIFICATION OF SUBJECT MATTER
Int.Cl[7] C07C69/734, C07C255/13, C07F7/08, A01N37/38

According to International Patent Classification (IPC) or to both national classification and IPC

B. FIELDS SEARCHED
Minimum documentation searched (classification system followed by classification symbols)
Int.Cl[7] C07C69/734, C07C255/13, C07F7/08, A01N37/38

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | EP, 472224, A1 (IMPERIAL CHEMICAL INDUSTRIES PLC), 26 February, 1992 (26.02.92) & JP, 63-216848, A & US, 5438059, A | 1~12 |
| A | US, 5286750, A (BASF Aktiengesellschaft), 15 February, 1994 (15.02.94) & JP, 3-157350, A & EP, 422597, A2 | 1~12 |
| A | US, 4822908, A (BASF Aktiengesellschaft), 18 May, 1989 (18.05.89) & JP, 1-131136, A & EP, 251082, A2 | 1~12 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 January, 2000 (07.01.00) | 18 January, 2000 (18.01.00) |
| Name and mailing address of the ISA/ Japanese Patent Office | Authorized officer |
| Facsimile No. | Telephone No. |

Form PCT/ISA/210 (second sheet) (July 1992)